# EUROPEAN PATENT APPLICATION

(11) **EP 3 124 463 A1**
(43) Date of publication of application: **01.02.2017**
(21) Application number: 15769437.3
(22) Date of filing: 25.03.2015
(51) Int. Cl.: C07C 41/06, C07C 43/184, C07B 61/00

(54) **METHOD FOR PRODUCING CYCLOPENTYL ALKYL ETHER COMPOUND**

(30) Priority: 28.03.2014 JP 2014069524
(71) Applicant: Zeon Corporation, Tokyo 100-8246 (JP); The University of Tokyo, Bunkyo-ku, Tokyo 113-8654 (JP)
(72) Inventor: NAKANO, Keita, Tokyo 100-8246 (JP); SUGAWARA, Mitsuru, Tokyo 100-8246 (JP); KOBAYASHI, Shu, Tokyo 113-8654 (JP)
(74) Representative: Beckmann, Claus
(86) International application number: PCT/JP2015/059071
(87) International publication number: WO 2015/147035

(57) **Abstract**

The present invention is a method for producing a cyclopentyl alkyl ether compound comprising reacting substituted or unsubstituted cyclopentene with an alcohol compound represented by a formula (2): R¹OH in the presence of an acidic zeolite, the cyclopentyl alkyl ether compound being represented by a formula (1): R¹-O-R², wherein R¹ represents a substituted or unsubstituted alkyl group having 1 to 10 carbon atoms, or a substituted or unsubstituted cycloalkyl group having 3 to 8 carbon atoms, and R² represents a substituted or unsubstituted cyclopentyl group. The present invention provides a method that can produce a cyclopentyl alkyl ether with high reaction efficiency through a liquid-phase reaction even when the raw material feed rate (amount) is increased.

## Description

### TECHNICAL FIELD

The present invention relates to a method for industrially advantageously producing a cyclopentyl alkyl ether compound that is useful as a solvent for washing (cleaning) electronic parts and precision machinery parts, a chemical reaction solvent, an extraction solvent, a crystallization solvent, an eluent used for chromatography, a solvent used for electronic/electrical materials, a release agent, and the like.

### BACKGROUND ART

A method that subjects an olefin and an alcohol to an addition reaction in the presence of a solid acid catalyst to produce an ether has been known.

For example, Patent Literature 1 discloses a method for producing cyclopentyl methyl ether that utilizes an acidic ion-exchange resin having a water content of 5 wt% or less as a catalyst. According to the method disclosed in Patent Literature 1, however, since it is necessary to effect the reaction in a gas phase in order to suppress deterioration in the acidic ion-exchange resin, the reaction yield is low.

Patent Literature 2 discloses a method for producing methyl t-butyl ether that utilizes a crystalline aluminosilicate as a catalyst. Patent Literature 3 discloses a method for producing cyclohexyl methyl ether that utilizes a special aluminosilicate having a large number of outer-surface acid sites as a catalyst. Patent Literature 4 discloses a method for producing cyclohexyl methyl ether that utilizes tungsten oxide having a specific crystal water content as a catalyst.

However, Patent Literature 2 to 4 are silent about the production of cyclopentyl methyl ether.

Patent Literature 4 states that, when a high-silica zeolite (H-ZSM-5) was used when reacting cyclohexene and methanol, the yield of methyl cyclohexyl ether was as low as 3.7%.

### CITATION LIST

### PATENT LITERATURE

Patent Literature 1: WO2003/2500
Patent Literature 2: JP-A-59-25345
Patent Literature 3: JP-A-61-249945
Patent Literature 4: JP-A-5-163188

### SUMMARY OF INVENTION

### TECHNICAL PROBLEM

The invention was conceived in view of the above situation. An object of the invention is to provide a method that can produce a cyclopentyl alkyl ether with high reaction efficiency through a liquid-phase reaction even when the raw material feed rate (amount) is increased.

### SOLUTION TO PROBLEM

The inventors conducted extensive studies in order to solve the above problem. As a result, the inventors found that it is possible to stably produce a cyclopentyl alkyl ether with high reaction efficiency even when the raw material feed rate (amount) is increased, by reacting cyclopentene or a derivative thereof and an alcohol compound in a liquid state in the presence of an acidic zeolite, for example. This finding has led to the completion of the invention.

One aspect of the invention provides the following method for producing a cyclopentyl alkyl ether compound (see (1) to (4)).
[1] A method for producing a cyclopentyl alkyl ether compound including reacting substituted or unsubstituted cyclopentene with an alcohol compound represented by the formula (2): R¹OH in the presence of an acidic zeolite, the cyclopentyl alkyl ether compound being represented by the formula (1): R¹-O-R²,
   wherein R¹ represents a substituted or unsubstituted alkyl group having 1 to 10 carbon atoms, or a substituted or unsubstituted cycloalkyl group having 3 to 8 carbon atoms, and R² represents a substituted or unsubstituted cyclopentyl group.
[2] The method for producing a cyclopentyl alkyl ether compound according to [1], wherein R¹ in the formula (1) is an alkyl group having 1 to 10 carbon atoms.
[3] The method for producing a cyclopentyl alkyl ether compound according to [1] or [2], wherein the acidic zeolite is an H-ZSM-5-type zeolite.
[4] The method for producing a cyclopentyl alkyl ether compound according to any one of [1] to [3], wherein the reaction is effected using a flow method.

### ADVANTAGEOUS EFFECTS OF INVENTION

The method for producing a cyclopentyl alkyl ether compound according to one aspect of the invention can produce a cyclopentyl alkyl ether with high reaction efficiency through a liquid-phase reaction even when the raw material feed rate (amount) is increased.

The method for producing a cyclopentyl alkyl ether compound according to one aspect of the invention can industrially advantageously produce the desired cyclopentyl alkyl ether compound.

### BRIEF DESCRIPTION OF DRAWINGS

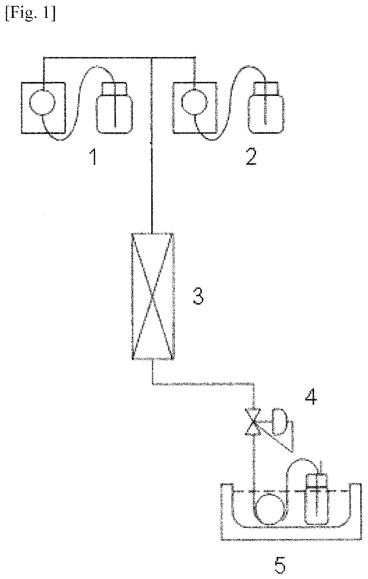

FIG. 1 is a schematic view illustrating a reactor that is used to implement a production method according to one embodiment of the invention.

### DESCRIPTION OF EMBODIMENTS

The exemplary embodiments of the invention are described in detail below.

According to one embodiment of the invention, a method for producing a cyclopentyl alkyl ether compound (hereinafter may be referred to as "production method") includes reacting substituted or unsubstituted cyclopentene with an alcohol compound represented by the formula (2): R¹OH (hereinafter may be referred to as "alcohol compound (2)") in the presence of an acidic zeolite, the cyclopentyl alkyl ether compound being represented by the formula (1): R¹-O-R². Note that the expression "substituted or unsubstituted" used herein in connection with a group or the like means that the group or the like is unsubstituted, or substituted with a substituent.

The production method according to one embodiment of the invention reacts substituted or unsubstituted cyclopentene with the alcohol compound (2).

A substituent that may substitute cyclopentene (that is substituted or unsubstituted) used in connection with one embodiment of the invention is not particularly limited as long as the substituent is a group that is inert under the reaction conditions. Note that the term "substituted or unsubstituted cyclopentene" used herein may be hereinafter referred to as "cyclopentene or a derivative thereof". Examples of the substituent include an alkyl group having 1 to 4 carbon atoms (e.g., methyl group, ethyl group, n-propyl group, isopropyl group, n-butyl group, t-butyl group, sec-butyl group, and isobutyl group); an alkoxy group having 1 to 4 carbon atoms (e.g., methoxy group, ethoxy group, n-propoxy group, sec-propoxy group, n-butoxy group, t-butoxy group, and sec-butoxy group); an alkylthio group having 1 to 4 carbon atoms (e.g., methylthio group, ethylthio group, n-propylthio group, sec-butylthio group, and t-butylthio group); a halogen atom (e.g., fluorine atom, chlorine atom, bromine atom, and iodine atom); an aryl group (e.g., phenyl group); and the like.

Specific examples of cyclopentene or a derivative thereof include cyclopentene; an alkylcyclopentene such as 1-methylcyclopentene, 2-methylcyclopentene, 3-methylcyclopentene, 3-ethylcyclopentene, 3-sec-butylcyclopentene, 2-t-butylcyclopentene, and 1,3-dimethylcyclopentene; an alkoxycyclopentene such as 3-methoxycyclopentene, 3-ethoxycyclopentene, 2-sec-butoxycyclopentene, and 3-t-butoxycyclopentene; an alkylthiocyclopentene such as 3-methylthiocyclopentene, 3-ethylthiocyclopentene, 2-sec-butylthiocyclopentene, and 3-t-butylthiocyclopentene; a halogenated cyclopentene such as 1-fluorocyclopentene, 2-chlorocyclopentene, 3-chlorocyclopentene, 2-bromocyclopentene, and 3-bromocyclopentene; an arylcyclopentene such as 1-phenylcyclopentene; and the like.

The alcohol compound (2) used in connection with one embodiment of the invention is a compound represented by the formula (2): R¹OH. Note that R¹ represents a substituted or unsubstituted alkyl group having 1 to 10 carbon atoms, or a substituted or unsubstituted cycloalkyl group having 3 to 8 carbon atoms.

Examples of the alkyl group having 1 to 10 carbon atoms that forms the substituted or unsubstituted alkyl group having 1 to 10 carbon atoms include a methyl group, an ethyl group, an n-propyl group, an isopropyl group, an n-butyl group, a sec-butyl group, a t-butyl group, an n-pentyl group, a neopentyl group, an n-hexyl group, an n-heptyl group, an n-octyl group, an n-nonyl group, an n-decyl group, and the like.

Examples of the cycloalkyl group having 3 to 8 carbon atoms that forms the substituted or unsubstituted cycloalkyl group having 3 to 8 carbon atoms include a cyclopropyl group, a cyclobutyl group, a cyclopentyl group, a cyclohexyl group, a cycloheptyl group, a cyclooctyl group, and the like.

Examples of a substituent that may substitute the alkyl group having 1 to 10 carbon atoms (that is substituted or unsubstituted) include an alkoxy group having 1 to 10 carbon atoms, such as a methoxy group and an ethoxy group; an alkylthio group having 1 to 10 carbon atoms, such as a methylthio group and an ethylthio group; a halogen atom such as a fluorine atom, a chlorine atom, and a bromine atom; and the like.

Examples of a substituent that may substitute the cycloalkyl group having 3 to 8 carbon atoms (that is substituted or unsubstituted) include an alkyl group having 1 to 10 carbon atoms, such as a methyl group, an ethyl group, an n-propyl group, and an isopropyl group; an alkoxy group having 1 to 10 carbon atoms, such as a methoxy group and an ethoxy group; an alkylthio group having 1 to 10 carbon atoms, such as a methylthio group and an ethylthio group; a halogen atom such as a fluorine atom, a chlorine atom, and a bromine atom; and the like.

Examples of the substituted cycloalkyl group having 3 to 8 carbon atoms include a cycloalkyl group that is substituted with an alkoxy group having 1 to 10 carbon atoms, such as a 2-methoxycyclopropyl group and a 3-ethoxycyclohexyl group; a cycloalkyl group that is substituted with an alkylthio group having 1 to 10 carbon atoms, such as a 2-methylthiocyclopropyl group and a 3-ethylthiocyclohexyl group; a halogenated cycloalkyl group such as a 2-chlorocyclopropyl group and a 3-bromocyclohexyl group; and the like.

Among these, an alkyl group having 1 to 10 carbon atoms and a cycloalkyl group having 3 to 8 carbon atoms are preferable.

Specific examples of the alcohol compound (2) include the alcohol compound represented by the formula (2) wherein R¹ is an alkyl group having 1 to 10 carbon atoms, such as methanol, ethanol, n-propanol, isopropanol, n-butanol, sec-butanol, isobutanol, tert-butanol, n-pentanol, and n-hexanol; the alcohol compound represented by the formula (2) wherein R¹ is a substituted alkyl group having 1 to 10 carbon atoms, such as an alkoxyalkyl alcohol such as methoxymethyl alcohol, 1-methoxyethyl alcohol, 2-methoxyethyl alcohol, 2-ethoxy-tert-butyl alcohol, and 2-ethoxy-n-hexyl alcohol, an alkylthioalkyl alcohol such as methylthiomethyl alcohol, 1-methylthioethyl alcohol, 2-methylthio-tert-butyl alcohol, 3-methylthio-n-butyl alcohol, and 4-methylthio-n-hexyl alcohol, and a halogenated alkyl alcohol such as chloromethyl alcohol, bromomethyl alcohol, 1-chloroethyl alcohol, 2-chloro-n-propyl alcohol, 2-bromo-tert-butyl alcohol, 2-bromo-n-butyl alcohol, and 2-chloro-n-hexyl alcohol; the alcohol compound represented by the formula (2) wherein R¹ is a cycloalkyl group having 3 to 8 carbon atoms, such as cyclopropyl alcohol, cyclopenthyl alcohol, cyclohexyl alcohol, cycloheptyl alcohol, and cyclooctyl alcohol; the alcohol compound represented by the formula (2) wherein R¹ is a substituted cycloalkyl group having 3 to 8 carbon atoms, such as 2-chlorocyclopenthyl alcohol, 4-methoxycyclohexyl alcohol, and 3-methylthiocycloheptyl alcohol; and the like.

It is preferable to use cyclopentene and the alcohol compound represented by the formula (2) wherein R¹ is an alkyl group having 1 to 10 carbon atoms, since the advantageous effects of the invention can be more easily achieved.

In one embodiment of the invention, the acidic zeolite is used as the reaction catalyst.

The term "acidic zeolite" used herein refers to a zeolite that includes an H⁺ group or a Lewis acid site on the surface thereof.

A zeolite includes an SiO₄ tetrahedron and an AlO₄ tetrahedron. A number of types of zeolites that differ in the Si content ratio, the Al content ratio, and the bonding state of each tetrahedron are known. A zeolite has a three-dimensional framework structure in which pores are formed in the lattice. The size and the shape of the pores differ depending on the type of zeolite. It is known that the pores formed in a zeolite may have a pore size of 3 to 12 Å and a one-dimensional, two-dimensional, or three-dimensional shape.

A zeolite has an ion-exchange capacity. A zeolite normally includes an alkali metal ion (e.g., Na or K) in the framework. It is possible to easily effect an ion-exchange reaction by bringing a zeolite into contact with various cations.

The acidic zeolite used in connection with one embodiment of the invention is preferably an H-type zeolite produced from a β-type zeolite, a faujasite-type zeolite, a mordenite-type zeolite, an L-type zeolite, a Y-type zeolite, an omega-type zeolite, a ZSM-5-type zeolite, a ferrierite-type zeolite, or the like using the following method or the like, and more preferably an H-type zeolite (H-ZSM-5-type zeolite) produced from a ZSM-5-type zeolite.

For example, the H-type zeolite can be obtained by bringing a zeolite into contact with an ammonium ion aqueous solution (e.g., NH₄Cl or NH₄NO₃ aqueous solution) to obtain an ammonium ion-type zeolite, and calcining the ammonium ion-type zeolite at a temperature equal to or higher than 300°C to remove ammonia. The H-type zeolite can also be obtained by bringing a zeolite into contact with a strong acid (e.g., hydrochloric acid) to effect a direct ion-exchange reaction with the H ion.

A commercially-available product may be used directly as the acidic zeolite.

Cyclopentene or a derivative thereof and the alcohol compound (2) may be brought into contact with each other in an arbitrary way in the presence of the acidic zeolite. For example, cyclopentene or a derivative thereof and the alcohol compound (2) may be brought into contact with each other in the presence of the acidic zeolite using a method that adds the acidic zeolite to a mixture including cyclopentene or a derivative thereof and the alcohol compound (2) (hereinafter may be referred to as "mixture"), and stirs the resulting mixture (batch method), a method that charges a column with the acidic zeolite, and passes the mixture through the column (hereinafter referred to as "reaction column") (flow method), or the like. It is preferable to use the flow method from the viewpoint of work efficiency and capability to continuously purify the reaction product.

The mixture is prepared by mixing cyclopentene or a derivative thereof and the alcohol compound (2) in a given ratio. In this case, a mixture including cyclopentene or a derivative thereof and the alcohol compound (2) may be prepared in advance, stored in a tank, and fed to the reaction column from the tank, or cyclopentene or a derivative thereof and the alcohol compound (2) may be stored in different tanks, separately fed from the different tanks, and mixed immediately before being introduced into the reaction column.

When using the batch method, given amounts of the acidic zeolite, cyclopentene or a derivative thereof, and the alcohol compound (2) are added to a reactor, and the mixture is stirred at a given temperature under a given pressure. In this case, the acidic zeolite is normally used in a ratio of 0.01 to 200 parts by mass, preferably 0.1 to 150 parts by mass, and more preferably 1 to 100 parts by mass, based on 100 parts by mass of cyclopentene or a derivative thereof.

The reaction temperature is normally set to 50 to 250°C, and preferably 80 to 200°C. The reaction pressure is normally set to a value within the range from normal pressure (1013 hPa (hereinafter the same)) to 10 MPa, and preferably a value within the range from normal pressure to 5 MPa. Note that the reaction pressure is determined taking account of the reaction temperature and the like. The reaction time is determined taking account of the reaction scale and the like, but is normally set to 0.5 to 24 hours, and preferably 1 to 10 hours.

The reaction is preferably effected in an inert atmosphere (e.g., nitrogen atmosphere).

Cyclopentene or a derivative thereof and the alcohol compound (2) may be used in an arbitrary ratio. Note that it is preferable to use the alcohol compound (2) in excess with respect to cyclopentene or a derivative thereof. Since the mixture is heated for a long time when using the batch method, a polymer of cyclopentene or a derivative thereof may be produced if the mixture is reacted in a state in which cyclopentene or a derivative thereof is used in excess with respect to the alcohol compound (2). Cyclopentene or a derivative thereof and the alcohol compound (2) are normally used in a molar ratio (cyclopentene or derivative thereof/alcohol compound (2)) of 1/1 to 1/50, preferably 1/1 to 1/30, and more preferably 1/1 to 1/20.

When using the flow method, the mixture is passed (circulated) through the reaction column. In this case, a column provided with a heater (heating device) is used as the reaction column, and the mixture is passed through the reaction column heated to a given temperature (reaction temperature).

Specific examples of the method that brings cyclopentene or a derivative thereof and the alcohol compound (2) into contact with each other in the presence of the acidic zeolite using the flow method include a method that utilizes a reaction column 3 illustrated in FIG. 1 (alone) that is charged with the acidic zeolite. It is possible to further improve the conversion rate of cyclopentene or a derivative thereof (or the alcohol compound (2)) by utilizing a plurality of reaction columns in combination.

The size of the column is not particularly limited. A column having an appropriate size may be selected taking account of the reaction scale. When a plurality of reaction columns are used in combination, each column may be charged with an identical acidic zeolite, or may be charged with a different acidic zeolite.

The mixture may be passed through the reaction column charged with the acidic zeolite using a down-flow method that passes the mixture through the reaction column from the upper part of the reaction column, or an up-flow method that passes the mixture through the reaction column from the lower part of the reaction column. It is preferable to use the down-flow method since the desired product can be obtained with a higher conversion rate and higher selectivity. The mixture may be passed through the reaction column charged with the acidic zeolite in a gaseous state, a liquid state, or a gaseous-liquid state.

The mixture is normally passed through the reaction column under a pressure from normal pressure to 10 MPa, preferably from normal pressure to 5 MPa, and more preferably from normal pressure to 3 MPa.

The reaction temperature is normally set to 50 to 200°C, and preferably 80 to 180°C.

When using the flow method, cyclopentene or a derivative thereof and the alcohol compound (2) may be used in an arbitrary ratio. When using the flow method, since the mixture is heated for only a short time, cyclopentene or a derivative thereof is not polymerized. However, the amount of dialkyl ether (by-product) produced may increase if the alcohol compound (2) is used in excess with respect to cyclopentene or a derivative thereof. Cyclopentene or a derivative thereof and the alcohol compound (2) are normally used in a molar ratio (cyclopentene or derivative thereof/alcohol compound (2)) of 1/5 to 20/1, preferably 1/4 to 10/1, more preferably 1/3 to 5/1, and still more preferably 1/3 to 3/1.

Cyclopentene or a derivative thereof and the alcohol compound (2) are normally passed through the reaction column at a space velocity (i.e., a value (hr⁻¹) that represents a volume processed per unit time with respect to the catalyst volume) of 0.01 to 100 hr⁻¹, and preferably 0.1 to 30 hr⁻¹.

When a plurality of reaction columns are used, the reaction temperature, the flow rate, and the like may be changed on a reaction column basis.

After completion of the reaction, the desired cyclopentyl alkyl ether compound is isolated from the reaction mixture using a normal separation-purification method (e.g., extraction with a solvent, or distillation). Note that distillation may be performed a plurality of times.

A known distillation apparatus (e.g., a continuous rectification apparatus provided with a rectifying column) may be used for distillation, for example.

After passing the mixture through the reaction column charged with the acidic zeolite, the resulting reaction mixture may be passed through the reaction column again, and may be continuously distilled using a distillation apparatus provided with a Raschig ring, for example. In this case, unreacted cyclopentene or derivative thereof and the alcohol compound (2) can be returned to the reaction column, and reacted again. Therefore, it is possible to obtain the desired product with a higher conversion rate.

The reaction may be effected in the absence of a solvent, or may be effected in an inert solvent that dissolves cyclopentene or a derivative thereof (raw material), and is immiscible with water.

Examples of the solvent include a saturated aliphatic hydrocarbon such as n-butane, n-pentane, n-hexane, n-heptane, n-octane, n-nonane, and n-decane; an aromatic hydrocarbon such as benzene, toluene, ethylbenzene, xylene, anisole, cumene, and nitrobenzene; a saturated alicyclic hydrocarbon such as cyclopentane, an alkyl-substituted cyclopentane, an alkoxy-substituted cyclopentane, a nitro-substituted cyclopentane, cyclohexane, an alkyl-substituted cyclohexane, an alkoxy-substituted cyclohexane, a nitro-substituted cyclohexane, cycloheptane, an alkyl-substituted cycloheptane, an alkoxy-substituted cycloheptane, a nitro-substituted cycloheptane, cyclooctane, an alkyl-substituted cyclooctane, an alkoxy-substituted cyclooctane, and a nitro-substituted cyclooctane; nitrogen; argon; air; helium; and the like.

The solvent may be used in an arbitrary amount as long as the reaction is not hindered. The solvent is normally used in a ratio of 10 to 90 vol%, and preferably 20 to 80 vol%, based on the total amount of the reaction mixture (reactant).

The above operation makes it possible to produce the desired cyclopentyl alkyl ether represented by the formula (1): R¹-O-R² while suppressing deterioration in catalytic activity with the passing of time, and achieving high reaction efficiency even when the raw material feed rate (amount) is increased.

Note that R² in the formula (1) is the same as defined above, and R¹ in the formula (1) is a substituted or unsubstituted cyclopentyl group derived from cyclopentene or a derivative thereof.

More specifically, R¹ is a cyclopentyl group, an alkylcyclopentyl group (e.g., 2-methylcyclopentyl group or 3-sec-butylcyclopentyl group), an alkoxycyclopentyl group (e.g., 3-methoxycyclopentyl group or 2-sec-butoxycyclopentyl group), an alkylthiocyclopentyl group (e.g., 3-methylthiocyclopentyl group or 2-sec-butylthiocyclopentyl group), a halogenated cyclopentyl group (e.g., 2-chlorocyclopentyl group or 3-chlorocyclopentyl group), or the like.

### EXAMPLES

The invention is further described below by way of examples. Note that the invention is not limited to the following examples.

The content of each compound was measured using the following equipment under the following conditions.
Device: GC-2010 manufactured by Shimadzu Corporation
Column: DB-1 (length: 60 m, inner diameter 0.25 mm, thickness: 0.25 µm)
Column temperature: The column was heated from 50°C to 100°C at a rate of 5°C/min, heated from 100°C to 200°C at a rate of 10°C/min, heated from 200°C to 300°C at a rate of 20°C/min, and held at 300°C for 5 minutes
Inlet temperature: 250°C
Carrier gas: helium (flow rate: 1.0 mL/min)
Detector: FID
Detector temperature: 300°C

### Example 1

A 30 mL stainless steel autoclave was charged with 3.40 g (50 mmol) of cyclopentene, 1.60 g (50 mmol) of methanol, and 0.34 g of an H-ZSM-5-type zeolite (manufactured by JGC Catalysts and Chemicals Ltd.). The autoclave was sealed, and pressurized to 0.5 MPa using nitrogen. The mixture in the autoclave was reacted at 120°C for 5 hours while stirring the mixture using a magnetic stirrer. After completion of the reaction, the reaction mixture was held at 0°C for 1 hour, and the autoclave was opened. After the addition of anisole to the reaction mixture, the H-ZSM-5-type zeolite was filtered off using a filter having a pore size of 0.5 µm, and the filtrate was analyzed by gas chromatography. It was found that cyclopentyl methyl ether was produced in a yield of 12.3% with respect to the raw material (cyclopentene). The unreacted cyclopentene collection rate was 70.9%, and the methanol collection rate was 83.3%.

### Example 2

A reaction was effected substantially in the same manner as in Example 1, except that the reaction temperature was set to 150°C. It was found that cyclopentyl methyl ether was produced in a yield of 37.8% with respect to the raw material (cyclopentene). The unreacted cyclopentene collection rate was 62.2%, and the methanol collection rate was 52.7%.

### Example 3

A reaction was effected substantially in the same manner as in Example 2, except that 4.80 g (150 mmol) of methanol was used. It was found that cyclopentyl methyl ether was produced in a yield of 61.0% with respect to the raw material (cyclopentene). The unreacted cyclopentene collection rate was 35.0%, and the methanol collection rate was 70.9%.

### Example 4

A reaction was effected substantially in the same manner as in Example 3, except that the reaction temperature was set to 180°C. It was found that cyclopentyl methyl ether was produced in a yield of 63.2% with respect to the raw material (cyclopentene). The unreacted cyclopentene collection rate was 32.3%, and the methanol collection rate was 66.5%.

### Example 5

The following experiment was performed using the reactor illustrated in FIG. 1. In FIG. 1, reference numeral 1 indicates a tank for cyclopentene or a derivative thereof, reference numeral 2 indicates a tank for the alcohol compound (2), reference numeral 3 indicates a stainless steel reaction column (diameter: 10 mm, length: 100 mm), reference numeral 4 indicates a back-pressure regulator, and reference numeral 5 indicates an ice bath.

The reaction column 3 was charged with 3.66 g of an H-ZSM-5-type zeolite, and the discharge pressure of the back-pressure regulator 4 was set to 1.15 MPa and it was provided to the outlet of the reaction column. Cyclopentene was fed to the reaction column 3 from the tank 1 in an amount of 3.36 mL (36.5 mmol) per hour, and methanol was fed to the reaction column 3 from the tank 2 in an amount of 4.44 mL (109.6 mmol) per hour. After confirming that the raw material was discharged from the outlet of the back-pressure regulator, the temperature of the reaction column was increased to 150°C to effect a reaction. The outlet of the back-pressure regulator 4 was cooled to 0°C to collect the product. After the addition of anisole, the mixture was analyzed by gas chromatography. It was found that the reaction became stable within 1 hour from the start of the reaction, and cyclopentyl methyl ether was produced in a yield of 55.1% (on average (per hour) during the reaction (5 hours)) with respect to the raw material (cyclopentene). The unreacted cyclopentene collection rate was 40.3%, and the methanol collection rate was 65.6%.

### Example 6

A reaction was effected substantially in the same manner as in Example 5, except that the discharge pressure of the back-pressure regulator 4 was set to 2.2 MPa, cyclopentene was fed to the reaction column 3 in an amount of 10.08 mL (109.5 mmol) per hour instead of 3.36 mL (36.5 mmol) per hour, and methanol was fed to the reaction column 3 in an amount of 13.32 mL (328.8 mmol) per hour instead of 4.44 mL (109.6 mmol) per hour. It was found that cyclopentyl methyl ether was produced in a yield of 68.3% (on average (per hour) during the reaction (5 hours)) with respect to the raw material (cyclopentene). The unreacted cyclopentene collection rate was 30.3%, and the methanol collection rate was 68.0%.

### Comparative Example 1

A reaction was effected substantially in the same manner as in Example 2, except that hydrotalcite (manufactured by Wako Pure Chemical Industries, Ltd.) was used as the catalyst instead of the H-ZSM-5-type zeolite. It was found that cyclopentyl methyl ether was produced in a yield of 0.1% with respect to the raw material (cyclopentene). The unreacted cyclopentene collection rate was 99.9%, and the methanol collection rate was 95.0%.

### Comparative Example 2

A reaction was effected substantially in the same manner as in Example 2, except that montmorillonite (manufactured by Wako Pure Chemical Industries, Ltd.) was used as the catalyst instead of the H-ZSM-5-type zeolite. It was found that cyclopentyl methyl ether was produced in a yield of 0.7% with respect to the raw material (cyclopentene). The unreacted cyclopentene collection rate was 98.9%, and the methanol collection rate was 91.5%.

### Comparative Example 3

A reaction was effected substantially in the same manner as in Example 2, except that a synthetic zeolite A-4 (manufactured by Wako Pure Chemical Industries, Ltd.) was used as the catalyst instead of the H-ZSM-5-type zeolite. It was found that cyclopentyl methyl ether was produced in a yield of 0.1% with respect to the raw material (cyclopentene). The unreacted cyclopentene collection rate was 96.1%, and the methanol collection rate was 89.6%.

The reaction catalyst used in Examples 1 to 6 and Comparative Examples 1 to 3, the molar ratio of the raw material (cyclopentene (CP)) to methanol, the reaction temperature, the reaction method, the isolated yield of cyclopentyl methyl ether (CPME) with respect to the raw material (cyclopentene (CP)), the unreacted cyclopentene (CP) collection rate, and the methanol collection rate are listed in Table 1.

**TABLE 1**

| | Example 1 | Example 2 | Example 3 | Example 4 | Example 5 | Example 6 |
|---|---|---|---|---|---|---|
| Catalyst | H-ZSM-5 | H-ZSM-5 | H-ZSM-5 | H-ZSM-5 | H-ZSM-5 | H-ZSM-5 |
| CP:methanol (molar ratio) | 1:1 | 1:1 | 1:3 | 1:3 | 1:3 | 1:3 |
| Reaction temperature (°C) | 120 | 150 | 150 | 180 | 150 | 150 |
| Reaction method | Batch method | Batch method | Batch method | Batch method | Flow method | Flow method |
| Isolated yield (%) of CPME | 12.3 | 37.8 | 61.0 | 63.2 | 55.1 | 68.3 |
| Unreacted CP collection rate (%) | 70.9 | 62.2 | 35.0 | 32.3 | 40.3 | 30.3 |
| Methanol collection rate (%) | 83.3 | 52.7 | 70.9 | 66.5 | 65.6 | 68.0 |

| | | Comparative Example 1 | | Comparative Example 2 | | Comparative Example 3 |
|---|---|---|---|---|---|---|
| Catalyst | | Hydrotalcite | | Montmorillonite | | Synthetic zeolite A-4 |
| CP:methanol (molar ratio) | | 1:1 | | 1:1 | | 1:1 |
| Reaction temperature (°C) | | 150 | | 150 | | 150 |
| Reaction method | | Batch method | | Batch method | | Batch method |
| Isolated yield (%) of CPME | | 0.1 | | 0.7 | | 0.1 |
| Unreacted CP collection rate (%) | | 99.9 | | 98.9 | | 96.1 |
| Methanol collection rate (%) | | 95.0 | | 91.5 | | 89.6 |

As is clear from Table 1, the isolated yield of CPME was significantly high when the H-ZSM-5-type zeolite was used as the catalyst (Examples 1 to 6) as compared with the case where the H-ZSM-5-type zeolite was not used as the catalyst (Comparative Examples 1 to 3).

### REFERENCE SIGNS LIST

1: Tank for cyclopentene or derivative thereof
2: Tank for alcohol compound (2)
3: Reaction column
4: Back-pressure regulator
5: Ice bath

## Claims

1. A method for producing a cyclopentyl alkyl ether compound comprising reacting substituted or unsubstituted cyclopentene with an alcohol compound represented by a formula (2): R¹OH in the presence of an acidic zeolite, the cyclopentyl alkyl ether compound being represented by a formula (1): R¹-O-R²,
wherein R¹ in the formulas (1) and (2) represents a substituted or unsubstituted alkyl group having 1 to 10 carbon atoms, or a substituted or unsubstituted cycloalkyl group having 3 to 8 carbon atoms, and R² represents a substituted or unsubstituted cyclopentyl group.

2. The method for producing a cyclopentyl alkyl ether compound according to claim 1, wherein R¹ in the formula (1) is an alkyl group having 1 to 10 carbon atoms.

3. The method for producing a cyclopentyl alkyl ether compound according to claim 1 or 2, wherein the acidic zeolite is an H-ZSM-5-type zeolite.

4. The method for producing a cyclopentyl alkyl ether compound according to any one of claims 1 to 3, wherein the reaction is effected using a flow method.
